# EUROPEAN PATENT APPLICATION

(11) **EP 4 792 801 A1**
(43) Date of publication of application: **19.08.2026**
(21) Application number: 25157840.7
(22) Date of filing: 13.02.2025
(51) Int. Cl.: A61K 8/42, A61K 8/60, A61K 8/86, A61K 8/21, A61K 8/19, A61K 8/25, A61K 8/29, A61K 8/34, A61K 8/365, A61K 8/44, A61K 8/49, A61K 8/55, A61K 8/73, A61K 8/81, A61Q 11/00

(54) **NOVEL DENTRIFICE COMPOSITION**

(71) Applicant: Haleon UK IP Limited, Weybridge, Surrey KT13 0NY (GB)
(72) Inventor: The designation of the inventor has not yet been filed
(74) Representative: Haleon Patent Department

(57) **Abstract**

A dentifrice composition comprising:

a) water, between 20 % and 40 % by weight,

b) lactic acid or a salt thereof, between about 0.1 % and about 5 % by weight,

c) a copolymer of methyl vinyl ether (MVE) with maleic anhydride or acid, between about 0.1 % and about 0.5 % by weight,

d) sodium fluoride

e) calcium glycerophosphate and sodium glycerophosphate between about 0.01 % and about 0.50 % by weight;

and wherein the dentifrice composition has a slurry pH in the range from greater than about 5.5 to less than about 6.5.

Said composition for use in protecting teeth against dental erosion and for use in protecting teeth against dental caries.

## Description

### FIELD OF THE INVENTION

The present invention relates to a dentifrice composition for strengthening and protecting enamel of natural teeth, thereby providing protection against acidic challenges. A composition according to the invention comprises a particular carboxylic acid or alkali metal salt thereof, a source of free fluoride ions , a copolymer of methyl vinyl ether (MVE) with maleic anhydride or acid, and a metal salt (sodium and calcium) of glycerophosphate. Importantly the dentifrice composition is mildly acidic, having a slurry pH in the range greater than 5.5 to less than 6.5.

### BACKGROUND OF THE INVENTION

Tooth mineral is composed predominantly of calcium hydroxyapatite, Ca₁₀(PO₄)₆(OH)₂, which may be partially substituted with anions such as carbonate or fluoride, and cations such as zinc or magnesium. Tooth mineral may also contain non-apatitic mineral phases such as octacalcium phosphate and calcium carbonate.

Tooth decay may occur as a result of dental caries, which is a multifactorial disease where bacterial acids such as lactic acid produced by metabolism of dietary sugars leads to sub-surface demineralisation that does not fully remineralise in between sugar exposures, resulting in progressive tissue loss and eventually cavity formation. The presence of a plaque biofilm is a prerequisite for dental caries, and acidogenic bacteria such as *Streptococcus mutans* may become pathogenic when levels of sugars (i.e. easily fermentable carbohydrate such as sucrose), are elevated for extended periods of time.

Even in the absence of a plaque biofilm, loss of dental hard tissues can occur as a result of acid erosion and/or physical tooth wear; these processes are believed to act synergistically. Exposure of the dental hard tissues to acid can cause demineralisation, resulting in surface softening and a decrease in mineral density. This softened mineral is vulnerable to wear from physical contact. Under normal physiological conditions, partially demineralised tissues self-repair through the remineralising effects of saliva. Saliva is supersaturated with respect to calcium and phosphate, and in healthy individuals, saliva secretion serves to wash out the acid challenge, and to raise the pH so as to alter the equilibrium in favour of mineral deposition.

Dental erosion (i.e. acid erosion or acid wear) is a surface phenomenon that involves demineralisation, and ultimately complete dissolution of the tooth surface by acids that are not of bacterial origin. Most commonly the acid will be of dietary origin, such as citric acid from fruit or carbonated drinks, phosphoric acid from cola drinks and acetic acid such as from vinaigrette. Dental erosion may also be caused by repeated contact with hydrochloric acid (HCl) produced by the stomach, which may enter the oral cavity through an involuntary response such as gastroesophageal reflux, or through an induced response as may be encountered in sufferers of bulimia.

Tooth wear (i.e. physical tooth wear) is caused by attrition and/or abrasion. Attrition occurs when tooth surfaces rub against each other, a form of two-body wear. An often-dramatic example is that observed in subjects with bruxism, a tooth-grinding habit during sleep where the applied forces are high, and is characterised by accelerated wear, particularly on the occlusal surfaces. Abrasion typically occurs as a result of three-body wear, and the most common example is that associated with brushing with a toothpaste. In the case of fully mineralised enamel, levels of wear caused by commercially available toothpastes are minimal and of little or no clinical consequence. However, if enamel has been demineralised and softened by exposure to an erosive challenge, the enamel becomes more susceptible to wear. Enamel is thinnest at its junction with the dentine, which in health is located just below the gum margin. However, gum recession (especially associated with ageing) can expose the enamel-dentine junction and wear of enamel in this region can expose dentine, leading to hypersensitivity, as described below.

Dentine is a vital tissue that in vivo is normally covered by enamel or cementum depending on the location i.e. crown versus root respectively. Dentine has a much higher organic content than enamel and its structure is characterised by the presence of fluid-filled tubules that run from the surface of the dentine-enamel or dentine-cementum junction to the pulp interface. Dentine is much softer than enamel and consequently is more susceptible to wear. Subjects with exposed dentine should avoid the use of highly abrasive toothpastes. Again, softening of dentine by an erosive challenge will increase susceptibility of the tissue to wear. It is widely accepted that the origins of dentine hypersensitivity relate to changes in fluid flow in exposed tubules, (the hydrodynamic theory), that result in stimulation of mechanoreceptors thought to be located close to the pulp interface. Not all exposed dentine is sensitive since it is generally covered with a smear layer; an occlusive mixture comprised predominantly of mineral and proteins derived from dentine itself, but also containing organic components from saliva. Over time, the lumen of the tubule may become completely occluded with mineralised tissue. The formation of reparative dentine in response to trauma or chemical irritation of the pulp is also well-documented. Nonetheless, an erosive challenge can remove the smear layer and tubule "plugs" releasing dentinal fluid flow, making the dentine much more susceptible to external stimuli such as hot, cold and pressure. As previously indicated, an erosive challenge can also make the dentine surface much more susceptible to wear. In addition, dentine hypersensitivity worsens as the diameter of the exposed tubules increases, and since the tubule diameter increases as one proceeds in the direction of the pulp interface, progressive dentine wear can result in an increase in hypersensitivity, especially in cases where dentine wear is rapid.

Erosion and/or acid-mediated tooth wear are therefore primary aetiological factors in the development of dentine hypersensitivity.

It has been claimed that an increased intake of dietary acids, and a move away from formalised meal times, has been accompanied by a rise in the incidence of dental erosion and tooth wear in the populations of developed countries. In view of this, oral care compositions which can help prevent dental erosion and tooth wear and which provide protection from dental caries would be advantageous.

Oral care compositions often contain a source of fluoride ions for promoting remineralisation of teeth and for increasing the acid resistance of dental hard tissues. To be effective the fluoride ions must be available for uptake into the dental hard tissues being treated.

It has been observed that demineralised enamel will take up more fluoride from an acidic solution than from a neutral one (*e.g.* Friberger, The effect of pH upon fluoride uptake in intact enamel. Scand. J. Dent. Res. (1975) 83:339-344). The Friberger study investigated the *in vitro* uptake of fluoride from dentifrice slurries and from sodium fluoride solutions of different pH ranging from 7.1 to 4.5. The pH was adjusted with a few drops of 0.1 M HCl acid or NaOH. The investigation showed there was no significant difference between the agents (namely a sodium fluoride dentifrice, a potassium fluoride and manganese chloride dentifrice and a sodium fluoride solution of the same fluoride concentration), but that the effect of pH was significant. The uptake of fluoride in the form of fluorapatite was more than five times larger at the lower pH level.

GB 1,018,665 (Unilever Ltd) describes a fluoride dentifrice incorporating a water-soluble buffering system that comprise a weak organic acid and an alkali metal salt, for example acetic acid/sodium acetate and malic acid/sodium malate, and wherein the pH of a slurry of the dentifrice in simulated saliva is from 5 to 6. The dentifrice is disclosed as being capable of reducing enamel solubility compared to solutions at neutral pH.

US 2009/0087391A1 (Joziak) describes a foaming fluoride dental composition comprising a surface-active agent selected from the group consisting of non-ionic, zwitterionic or betaine surfactants or mixtures thereof and an acidifying agent in an amount sufficient to adjust the pH to 3 to 5. Suitable acidifying agents are organic acids such as malic acid, hydrosuccinic acid, citric acid and tartaric acids or mixtures thereof.

WO 01/66074 (Colgate) describes a dual-component dentifrice, one phase being alkaline and containing fluoride ions, the other phase being acidic and containing phosphate ions, which on mixing prior to use, provides an acidic phosphate fluoride composition (pH 4 to 6). It is suggested that the delivery of the dentifrice at an acidic pH can enhance the uptake of the fluoride ions into the tooth enamel.

WO 2006/133747 (in the name of Henkel) describes oral care compositions comprising low quantities (ie from 0.01 to 0.09 percent by weight of the composition) of various sparingly soluble rod-shaped nanoparticulate calcium compounds such as hydroxyapatite, fluorapatite or calcium fluoride. It is suggested that such compositions can also contain other calcium compounds, which need not be nanoparticulate, such as calcium glycerophosphate, or calcium containing abrasives such as chalk, calcium pyrophosphate or dicalcium phosphate dihydrate. Such compositions can also comprise a source of fluoride ions, preferably in an amount of 0.01 to 0.2 percent by weight.

US 4,363,794 (Lion Corporation) discloses an oral composition which comprises a stannous salt such as stannous fluoride, a water-soluble fluoride salt such as sodium fluoride and an orally acceptable acid such as L-ascorbic acid, lactic acid, malonic acid, tartaric acid, citric acid, hydrochloric acid and pyrophosphoric acid, the molar ratio of fluoride ion to stannous ion being in the range of 3.2 to 7:1, preferably 3.5 to 6:1, in an aqueous condition and the pH of the composition being in the range of from 2 to 4. The composition is disclosed as exhibiting excellent effects on the inhibition of dental caries. According to US 4, 363,794, the specified pH range results in an increase of effectiveness on the increment of acid-resistance for the treated enamel and on stability of the stannous ion. Low pH (below 2) tends to pose an obstacle to oral application of the composition whereas a pH above 4 often causes reduced availability and stability of stannous ion.

The use of fluoride-containing dentifrices formulated at substantially neutral pH, have also been described in the art for remineralizing and strengthening teeth. WO2006/1000071 (Glaxo Group Ltd) discloses dentifrice compositions that comprise, amongst other ingredients, a fluoride ion source, and have a pH in the range 6.5 to 7.5. Such compositions have been commercialized as SENSODYNE Pronamel toothpaste for use in protecting teeth against dietary acidic challenges.

In one aspect the present invention is based on the discovery that incorporation of a particular carboxylic acid(s) as described herein in a mildly acidic dentifrice composition comprising a source of fluoride ions, advantageously enhances the uptake of fluoride ions into dental enamel when compared to the same composition under a neutral pH, or when compared to the same mildly acidic composition but containing a different carboxylic acid (such as malic acid), or an inorganic acid (such as phosphoric acid).

In a further aspect the present invention is based on the discovery that incorporation of a copolymer of methyl vinyl ether with maleic anhydride or acid provides a further benefit of significantly increasing enamel solubility reduction without adversely impacting on uptake of fluoride.

In a yet further aspect, the present invention is based on the discovery that incorporation of a metal salt of glycerophosphate (preferably a calcium and / or sodium salt) provides a benefit of significantly increasing uptake of fluoride, with an associated benefit of improving enamel hardness.

The use of copolymers based on methyl vinyl ether and maleic acid in oral care compositions is known in the art. For example, US 4,485,090 discloses dentifrice compositions comprising a polymeric anionic membrane-forming material such as "Gantrez AN". According to US 4,485,090 the material attaches itself to tooth surfaces and forms a substantially continuous barrier thereon by complexing with calcium present in the teeth. The barrier formed is described as substantially reducing elution of a previously applied therapeutic agent (e.g. dental fluoride treatment), thereby prolonging the effectiveness of such agent. According to US 4,485,090, compositions of the invention therein need only be applied periodically (e.g. once daily) in order to achieve the desired reduction in elution and resultant control of caries and plaque.

Later-filed US patent application US2004/0146466 (Baig et al) discloses that particular polymeric mineral surface active agents such as synthetic anionic polymers e.g. polyacrylates and copolymers of maleic anhydride or acid and methyl vinyl ether (*e.g. Gantrez*), have a strong affinity for tooth enamel surface and that such polymers deposit a layer or coating on the enamel surface. Effective amounts of a polymeric mineral surface active agent are described as ranging from about 1% to about 35%, preferably from about 2 to about 30%, more preferably from about 5% to about 25%, and most preferably from about 6% to about 20% by weight of the total oral composition.

WO2007/069429 (Lion Corporation) discloses toothpaste compositions containing (A) from 0.3 to 1.2% by mass of at least one linear and water-soluble polyphosphate represented by the general formula Mₙ₊₂PₙO₃ₙ₊₁ (wherein M represents Na or K; and n is an integer of 2 or 3), (B) from 0.1% to 2.0% by mass of a methyl vinyl ether/maleic anhydride copolymer, a 2.0% by mass aqueous solution of which has a viscosity of from 5 to 1000 mPa.s at 25°C and pH 7.0, (C) from 0.6 to 2.0% by mass of a lauryl sulphate, and (D) from 0.2 to 1.0% by mass of a betaine type amphoteric surfactant, and the composition ratio by mass (C)/(D) ranging from 1 to 4. Such compositions are described as causing low irritation of the oral mucosa and as providing favourable foaming in use, as well as having an excellent effect on preventing the adhesion of stains to tooth surfaces.

WO2011/094499 (Colgate-Palmolive Company) discloses anti-erosion oral care formulations comprising a copolymer of a methyl vinyl ether and a maleic anhydride, such as Gantrez, and a metal compound or salt that becomes more soluble at acidic pH. According to WO2011/094499, a mucoadhesive polymer, such as Gantrez, may be incorporated into the orally acceptable vehicle in an amount ranging from 0.01 to 20% by weight, preferably 0.1 to 10% by weight and most preferably from 0.5 to 7% by weight of component. A "Low Polymer Formulation" and a "High Polymer Formulation" exemplified in WO2011/094499 comprises, respectively 0.5% and 2.0% by weight Gantrez.

A Technical Information Sheet, Bulletin VC-862A, published by Ashland Speciality Chemicals (Rev. 02-2015), reported that superior acid erosion resistance of enamel had been observed in an *in vitro* study, following pre-treatment of the enamel with a toothpaste containing 2% Gantrez S-97 polymer, and that the presence of the Gantrez was believed to be the primary reason for the improvement observed in reducing acid erosion.

WO2015/171836 (Procter & Gamble) describes oral care compositions containing 5% metal ions, at least 0.001% of stannous ions and optionally from about 0.001% to about 4% zinc ions; at least about 100ppm by weight of fluoride ions and at least about 0.03% by weight of a mineral surface active agent selected from, amongst others, copolymers of maleic anhydride or acid with methyl vinyl ether; at least 5% water; less than 10% by weight of a fused silica, calcium based abrasive and mixtures thereof, less than 5% of polyphosphates having n+3 or higher, wherein the weight ratio of total metal ion (stannous optionally zinc) is equal to or less than 0.5. WO2015/171836 discloses by appropriately balancing the ratio of total metal ions to a selected group of mineral surface active agents that fluoride uptake may be improved and specified benefits (antibacterial efficacy, fluoride uptake, demineralization and reduced stain) necessary to hit a "sweet spot" of oral care can be achieved in one composition. According to WO2015/171836, the compositions described therein provide remineralization enhancement and demineralization inhibition benefits by controlling deposition of surface protection agents, which when deposited in excess negatively impact fluoride uptake and remineralization of tooth lesions below the surface. The inclusion of a buffering agent is optional, and the oral compositions will typically have a pH from about 4 to about 7, preferably from about 4.5 to about 6.5 and more preferably from about 5 to about 6. WO2015/171836 discloses that the inclusion of Gantrez does not impact fluoride uptake from a NaF containing formula.

### SUMMARY OF THE INVENTION

In one aspect the present invention provides a dentifrice composition comprising
a) water, between 20 % and 40 % by weight
b) lactic acid or a salt thereof, between about 0.1 % and about 5 % by weight,
c) a copolymer of methyl vinyl ether (MVE) with maleic anhydride or acid, between about 0.1 % and about 0.5 % by weight,
d) sodium fluoride
e) calcium glycerophosphate and sodium glycerophosphate, between about 0.01 % and about 0.50 % in total, by weight;
and wherein the dentifrice composition has a slurry pH in the range from greater than about 5.5 to less than about 6.5.

Such compositions are of use in protecting teeth against dental erosion. Such compositions are also of use in protecting teeth against dental caries.

### BRIEF DESCRIPTION OF THE DRAWINGS

Figure 1: Effect of Calcium Glycerophosphate and Sodium Glycerophosphate on microhardness (VHN)
Figure 2: Effect of Calcium Glycerophosphate and Sodium Glycerophosphate on EFU
Figure 3: Effect of Calcium Glycerophosphate and Sodium Glycerophosphate on demineralization

### DETAILED DESCRIPTION OF THE INVENTION

A dentifrice composition according to the invention comprises calcium glycerophosphate and sodium glycerophosphate in an amount of (in total) between about 0.01 % and about 0.50 % by weight.

Calcium glycerophosphate is typically recognised as calcium 2,3-dihydroxypropan-2-yl phosphate (C₃H₇CaO₆P). Sodium glycerophosphate is typically recognised as disodium 2,3-dihydroxypropyl phosphate (C₃H₇Na₂O₆P). Other structural variants of either or both compounds may be possible (such as the 1,3-dihydroxypropyl and 1,2-dihydroxypropyl variants).

It will be understood that any of the following preferred ranges of amount of calcium glycerophosphate or sodium glycerophosphate; either when viewed individually or in any combination can be used to refer to the amount of calcium glycerophosphate or sodium glycerophosphate or mixtures thereof.

Preferably the dentifrice composition according to the invention comprises calcium glycerophosphate and sodium glycerophosphate in an amount of between about 0.02 % and about 0.23 % by weight, more preferably in an amount of between about 0.03 % and about 0.2 % by weight, more preferably in an amount of between about 0.04 % and about 0.18 % by weight, more preferably in an amount of between about 0.05 % and about 0.15 % by weight, more preferably in an amount of between about 0.06 % and about 0.14 % by weight, most preferably in an amount of between about 0.07 % and about 0.12 % by weight.

Alternatively, preferably the dentifrice composition comprises calcium glycerophosphate in an amount of about 0.01 % to about 0.1 % by weight and comprises sodium glycerophosphate in an amount of about 0.01 % to about 0.25 % by weight.

More preferably the dentifrice composition comprises calcium glycerophosphate in an amount of about 0.015 % to about 0.025 % by weight and comprises sodium glycerophosphate in an amount of about 0.02 % to about 0.07 % by weight; or wherein the calcium glycerophosphate comprises about 0.015 % to about 0.025 % and the sodium glycerophosphate comprises about 0.08 % to about 0.12 % by weight.

Most preferably the dentifrice composition comprises calcium glycerophosphate in an amount of about 0.02 % by weight and comprises sodium glycerophosphate in an amount of about 0.05 % by weight. Also, most preferably the dentifrice composition comprises calcium glycerophosphate in an amount of about 0.02 % by weight and comprises sodium glycerophosphate in an amount of about 0.1 % by weight.

Generally, the amount of sodium glycerophosphate present in the dentifrice composition is greater than the amount of calcium glycerophosphate present in the dentifrice composition.

In an alternative and / or additionally it is preferred that the ratio of calcium glycerophosphate and sodium glycerophosphate by weight (when both are present in the dentifrice composition) is from about 0.1 to about 10 to about 10 to about 0.1, more preferably is from about 0.1 to about 7 to about 7 to about 0.1, more preferably is from about 0.1 to about 5 to about 5 to about 0.1, more preferably is from about 0.1 to about 3 to about 3 to about 0.1, more preferably is from about 0.5 to about 3 to about 3 to about 0.5, more preferably is from about 0.75 to about 3 to about 3 to about 0.75, more preferably is from about 1 to about 3 to about 3 to about 1, and most preferably from about 1 to about 2.5.

Alternatively the ratio of calcium glycerophosphate and sodium glycerophosphate by weight (when both are present in the dentifrice composition) may be from about 0.2 to about 10 to about 10 to about 0.2, more preferably is from about 0.2 to about 7 to about 7 to about 0.2, more preferably is from about 0.2 to about 5 to about 5 to about 0.2, more preferably is from about 0.3 to about 5 to about 5 to about 0.3, more preferably is from about 0.4 to about 5 to about 5 to about 0.4, more preferably is from about 0.6 to about 5 to about 5 to about 0.6, more preferably is from about 0.8 to about 5 to about 5 to about 0.8, and most preferably from about 1 to about 5.

Other preferred ratios of calcium glycerophosphate and sodium glycerophosphate by weight (when both are present in the dentifrice composition) may be about 1:2 or about 1:5.

A composition according to the invention comprises a carboxylic acid or alkali metal salt thereof wherein the acid is selected from the group consisting of malonic acid, glutaric acid, tartaric acid, lactic acid and mixtures thereof. In one embodiment the carboxylic acid is lactic acid or an alkali metal salt thereof. Typical examples of suitable alkali metal salts include the sodium and potassium salts of the said carboxylic acids. In one embodiment the alkali metal salt is the potassium salt(s) of malonic, glutaric, tartaric, lactic acids and mixtures thereof. In one embodiment the alkali metal salt is selected from the sodium salt(s) of malonic, glutaric, tartaric, lactic acids and mixtures thereof. In one embodiment the carboxylic acid salt is potassium lactate. In one embodiment the carboxylic acid salt is sodium lactate.

The carboxylic acid or salt may be provided in the form of a solid or an aqueous solution, e.g. sodium lactate solution (60% w/w).

Suitably the carboxylic acid or alkali metal salt thereof is present in an amount of from 0.5% to 5.0% by weight of the total composition, for example from 1.0% to 4.5% or from 1.5% to 3.0% by weight of the total composition. A preferred amount is 2.0% by weight of the acid or 2.5% by weight of the salt.

A composition according to the invention comprises a source of free fluoride ions. Suitable examples of a source of free fluoride ions include an alkali metal fluoride such as sodium or potassium fluoride, polyvalent metal ion fluoride salts such as stannous fluoride, or salts of fluoride with cationic organic ions such as ammonium fluoride or bis-(hydroxyethyl)amino-propyl-N-hydroxyethyloctadecylamine-dihydrofluoride, (amine fluoride) or a mixture thereof in an amount to provide from 25 to 5000ppm of fluoride ions, preferably from 100 to 1500ppm. In one embodiment the source of free fluoride ions is stannous fluoride. In one embodiment the source of free fluoride ions is not stannous fluoride. In one embodiment the source of free fluoride ions is an alkali metal fluoride such as sodium fluoride. Suitably the composition contains from 0.05 % to 0.5 % by weight of sodium fluoride, e.g. 0.1 % by weight (equating to 450 ppm of fluoride ions), 0.205 % by weight (equating to 927ppm of fluoride ions), 0.2542 % by weight (equating to 1150 ppm of fluoride ions) or 0.3152% by weight (equating to 1426ppm of fluoride ions).

A composition according to the invention is mildly acidic i.e. has a slurry pH in the range from greater than 5.5 to less than 6.5, for example from pH 5.6 to 6.4, 5.7 to 6.3, or 5.8 to 6.2. In one embodiment the pH is about 6.2. The pH referred to is that measured when the dentifrice composition is slurried with water in a 1:3 weight ratio of the composition to water. Suitably the slurry is prepared by slurring the dentifrice composition with water in a weight ratio of one part dentifrice composition and three parts distilled water. The pH is determined using a standard pH meter.

Suitably a dentifrice composition of the invention comprises a pH modifying agent to adjust the pH of the composition to the desired pH. Suitable pH modifying agents include alkali metal hydroxides such as sodium hydroxide and potassium hydroxide, or inorganic acids such as hydrochloric acid or sulphuric acid. In one embodiment the pH modifying agent is sodium hydroxide. A pH modifying agent may be used in an amount from 0.005% to 5% by weight of the composition, such as from 0.01% to 2% or 0.02% to 1% by weight of the composition.

In one aspect a composition according to the invention comprises a surface protection agent which is a copolymer of methyl vinyl ether (MVE) with maleic anhydride or acid. In one embodiment the surface protection agent is a copolymer of MVE with maleic acid. In general, the copolymer is a linear copolymer comprising alternating units of MVE and maleic anhydride or acid. In one embodiment the copolymer comprises a 1:4 to 4:1 ratio of MVE : maleic anhydride or acid, such as a 1:1 ratio of MVE : maleic anhydride or acid i.e. the MVE content is about 50 mole % and the maleic anhydride or acid content is about 50 mole %. In one embodiment the copolymer is the acid form of a copolymer of MVE with maleic anhydride wherein the anhydride is fully or partially hydrolysed, e.g. following co-polymerization to provide the corresponding acid. In one embodiment the copolymer has a molecular weight in the range 100,000 to 2,000,000 *e.g.* from 500,000 to 1,900,000 or from 1,000,000 to 1,800,000.

Suitably a copolymer for use in the invention is available commercially under the trade name GANTREZ^{®} such as GANTREZ^{®} S-97 HSU solution (Mw 1,500,000), GANTREZ^{®} S-97 BF (Mw 1,200,000), GANTREZ^{®} S-96 (Mw 700,000) and GANTREZ^{®} S-95 (Mw 150,000), all of which are copolymers of MVE with maleic acid. In one embodiment the copolymer is GANTREZ^{®} S-97 which is a copolymer of MVE with maleic acid having an approximate molecular weight of 1,200,000 or 1,500,000. GANTREZ^{®} S-97 may be provided in the form of a solid (powder) or as a liquid such as an aqueous solution e.g. GANTREZ ^{®}S-97 HSU solution. In one embodiment the copolymer comprises a GANTREZ^{®} polymer with the following structure and below indicated properties: 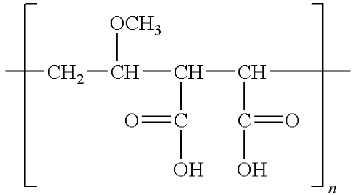 Di-basic acid with pKa₁ = 3.5, pK a₂ = 6.5

| Property | Gantrez S-97 BF | Gantrez S-97 HSU Solution |
|---|---|---|
| Appearance @ 25°C | White to off-white, free flowing powder | Slightly hazy viscous solution |
| % Solids (Active) | 94 | 15-17 |
| % Moisture | ≤6 | 85-83 |
| Approx. Molecular Weight | 1,200,000 | 1,500,000 |

Suitably the rheological properties of the copolymer can be modified by the addition of salts and bases. GANTREZ^{®} copolymers are available commercially from various sources including Ashland Speciality Chemicals, Bound Brook, N.J. 08805, USA and International Specialty Products, Wayne, NJ, USA.

Another commercially available source for the copolymer is available under the trade name of ORAREZ^{®}. These are supplied by BOAI. A particularly preferred source for the purposes of the present invention is ORAREZ W-100 L16.

It is a challenge to provide a dentifrice composition that delivers an enhanced fluoridation benefit when the composition comprises a surface protection agent (*i.e.* a copolymer of use in the invention as hereinabove defined). This is because of surface coverage of sites on the tooth surface by the agent where fluoridation typically takes place. Advantageously in the present invention, the copolymer can be combined with a source of fluoride ions without adversely impacting upon the delivery of fluoride to the dental enamel. It has now been unexpectedly discovered that a low amount of the copolymer provides an improvement with respect to enamel solubility reduction without significantly negatively impacting on fluoride uptake Accordingly, when present, the copolymer is used in an amount from 0.05 % to 2 % by weight of the composition, such as from 0.1 % to 1 % or from 0.15 % to 0.5 % or from 0.2 % to 0.4 % by weight of the composition. In one embodiment the copolymer is used in an amount of about 0.25% by weight of the composition. It has been surprisingly found in *in vitro* testing, reported herein, that when a low amount (from 0.2% to 0.3 % by weight, exemplified herein by about 0.25 % by weight) of copolymer is used, a significant improvement may be observed with respect inhibition of demineralization without adversely affecting fluoride uptake. These findings have been supported further by the findings of an *in-situ* erosion study, also reported herein, where a composition according to the invention comprising about 0.25 % by weight of a methyl vinyl ether maleic acid copolymer, was seen to outperform all other dentifrice compositions tested, with respect to fluoride uptake, remineralization enhancement and demineralization inhibition. In one embodiment the copolymer is used in amount of about 0.25 % by weight of the composition and the composition has a slurry pH of about 6.2.

In one embodiment a composition of the invention does not comprise stannous ions and/or zinc ions. For example, in one embodiment a composition of the invention does not comprise from about 0.001 % to about 5 % of metal ions wherein the metal ions comprise at least 0.001% of stannous ions and optionally from about 0.001 % to about 4 % of zinc ions. In one embodiment the composition does not comprise a metal compound or salt that becomes more soluble at acidic pH. In one embodiment the composition does not comprise a calcium or zinc compound or salt.

Compositions of the present invention may contain appropriate formulating agents such as dental abrasives, surfactants, thickening agents, humectants, flavouring agents, sweetening agents, opacifying or colouring agents, preservatives and water, selected from those conventionally used in the oral care composition art for such purposes.

Examples of suitable dental abrasives include silica abrasives such as those marketed under the following trade names Zeodent, Sident, Sorbosil or Tixosil by Huber, Degussa, Ineos and Rhodia respectively. The silica abrasive should be present in an amount sufficient to ensure adequate cleaning of teeth by the dentifrice whilst not promoting abrasion of teeth.

The silica abrasive is generally present in an amount up to 15 % by weight of the total composition, for example from 2 % to 10 % by weight, and preferably at least 5 % for example from 5 % to 7 % by weight, especially 6 % by weight of the total composition. Reducing the level of silica abrasive has the advantage of not only lowering the abrasivity of the dentifrice but also minimising any interaction of the abrasive with fluoride ions thereby increasing the availability of free fluoride ions.

Suitable surfactants for use in the present invention include amphoteric surfactants for example, long chain alkyl betaines, such as the product marketed under the tradename 'Empigen BB' by Albright & Wilson, and preferably long chain alkyl amidoalkyl betaines, such as cocamidopropylbetaine, or low ionic surfactants such as sodium methyl cocoyl taurate, which is marketed under the trade name Adinol CT by Croda, or mixtures thereof. An amphoteric surfactant can be used alone as sole surfactant or can be combined with a low ionic surfactant.

In one embodiment the surfactant is not a C₁₀₋₁₈ alkyl sulphate surfactant, such as sodium lauryl sulphate, commonly used in oral compositions.

Suitably, the surfactant is present in the range 0.1 % to 10 %, preferably 0.1 % to 5 % and more preferably 0.5 % to 1.5 % by weight of the total composition.

Suitable thickening agents include, for instance, nonionic thickening agents such as, for example, (C1-6)alkylcellulose ethers, for instance methylcellulose; hydroxy(C1-6)alkylcellulose ethers, for instance hydroxyethylcellulose and hydroxypropylcellulose; (C2-6)alkylene oxide modified (C1-6)alkylcellulose ethers, for instance hydroxypropyl methylcellulose; and mixtures thereof. Other thickening agents such as natural and synthetic gums or gum like material such as Irish Moss, xanthan gum, gum tragacanth, sodium carboxymethylcellulose, polyvinyl pyrrolidone, starch and thickening silicas may also be used. Preferably the thickening agent is mixture of a thickening silica and xanthan gum.

Advantageously the thickening agent is present in the range 0.1% to 30 %, preferably 1 % to 20 %, more preferably 5 % to 15 % by weight of the total composition.

Suitable humectants for use in compositions of the invention include for instance, glycerin, xylitol, sorbitol, propylene glycol or polyethylene glycol, or mixtures thereof; which humectant may be present in the range from 10% to 80%, preferably 20% to 60%, more preferably 25% to 50% by weight of the total composition.

A preferred opacifying agent is titanium dioxide which may be present in the range 0.05 % to 2 %, preferably 0.075 % to 0.2 %, for example 0.1 % by weight of the total composition. This amount enhances the visual appearance of the composition.

Flavouring agents that may be used in a composition of the invention include various flavouring aldehydes, esters, alcohols, and similar materials, as well as menthol, carvone and anethole as well as mixtures thereof. Examples of essential oils include spearmint, peppermint, wintergreen, sassafras, clove, sage, eucalyptus, marjoram, cinnamon, lemon, lime, grapefruit and orange. Suitably the flavouring agent may be used in an amount ranging from 0.01 % to 4 % such as 0.1 % to 3 % or 0.5 % to 2 % by weight of the composition.

Sweetening agents that may be used in a composition of the invention include, for example, sucrose, glucose, saccharin, sucralose, dextrose, levulose, lactose, mannitol, sorbitol, fructose, maltose, xylitol, saccharin salts (e.g. sodium saccharin) acesulfame and mixtures thereof. In one embodiment sodium saccharin is used as the sweetening agent. Suitably the sweetening agent may be used in an amount ranging from 0.005 % to 10 % such as 0.01 % to 3 % or 0.1 % to 1 % by weight of the composition.

Suitably dentifrice compositions of the present invention are aqueous dentifrice compositions. Water may make up the balance of the dentifrice composition. In one embodiment, the composition comprises 5 % to 80 % such as 10 % to 60 %, 15 % to 40 % or 20 % to 30 % by weight water. This amount of water includes the free water which is added plus that amount which is introduced with other components of the dentifrice composition, such as with sorbitol.

Dentifrice compositions of the present invention are typically formulated in the form of toothpastes or gels.

Additional oral care actives may be included in the compositions of the present invention.

Compositions of the present invention may further comprise a desensitising agent for combating dentine hypersensitivity. Examples of desensitising agents include a tubule blocking agent or a nerve desensitising agent and mixtures thereof, for example as described in WO 02/15809.

Suitable tubule blocking agents include strontium salts such as strontium chloride, strontium acetate or strontium nitrate. Suitably the strontium salt is used in an amount generally from 5% to 15% by weight of the composition.

In one embodiment the tubule blocking agent is an arginine calcium carbonate salt. Suitably the arginine salt is present in an amount ranging from 0.5 % to 30 % by weight of the composition, such as from 1 % to 10 % by weight of the composition or from 1 % to 10 % by weight of the composition such as from 2 % to 8 % by weight of the composition.

In one embodiment the tubule blocking agent is a bioactive glass. Suitably the bioactive glass consists of 45 % by weight silicon dioxide, 24.5 % by weight sodium oxide, 6 % by weight phosphorus oxide, and 24.5 % by weight calcium oxide. One such bioactive glass is available commercially under the trade name, NOVAMIN, also known as 4555 BIOGLASS. Suitably the bioactive glass is used in an amount generally from 1 % to 10 % by weight of the composition.

In one embodiment the tubule blocking agent is stannous fluoride. Stannous fluoride, through hydrolysis and oxidation reactions, forms insoluble metal salts that precipitate in dentinal tubules and on the dentine surface to provide effective relief from dentine hypersensitivity. Stannous fluoride may also be used to provide a source of fluoride capable of delivering protection from caries and plaque/gingivitis.

Suitable nerve desensitizing agents include potassium salts such as potassium citrate, potassium chloride, potassium bicarbonate, potassium gluconate and especially potassium nitrate. A desensitising amount of a potassium salt is generally between 2 % to 8 % by weight of the total composition, for example 5 % by weight of potassium nitrate can be used.

Compositions of the present invention may comprise a whitening agent, for example selected from a polyphosphate, e.g. sodium tripolyphosphate (STP) and/or any additional silica abrasive present may have high cleaning properties. STP may be present in an amount from 2 % to 15 %, for example from 5 % to 10 % by weight of the total composition.

Compositions of the present invention may comprise an oral malodour agent, for example a zinc salt such as zinc oxide or chloride.

The composition of the present invention is suitable for containing in and dispensing from an aluminium-plastic laminate tube or a plastic pump as conventionally used in the art.

Compositions of the present invention may be prepared by admixing the ingredients in the appropriate relative amounts in any order that is convenient and adjusting the pH to give a desired value.

An exemplary dentifrice composition according to the invention comprises:
an alkali metal salt of lactic acid such as sodium lactate in an amount from 0.5 % to 5.0 %;
a source of free fluoride ions such as sodium fluoride in an amount from 0.05 % to 0.5 %;
a copolymer of MVE with maleic anhydride or acid such as GANTREZ^{®} S-97 in an amount from 0.05% to 2 %; calcium glycerophosphate or sodium glycerophosphate or mixtures thereof, between about 0.01 % and about 0.25 % by weight; and wherein the composition has a slurry pH in the range greater than 5.5 to less than 6.5.

The present invention provides a composition as hereinbefore defined for use in protecting teeth against dental erosion. The present invention further provides a composition as hereinbefore defined for use in protecting teeth against dental caries.

The present invention provides a composition as hereinbefore defined for use in the treatment and/or inhibition of dental erosion on a dental surface. The present invention provides a composition as hereinbefore defined for use in the treatment and/or inhibition of caries on a dental surface.

The present invention also provides a method for protecting teeth against dental erosion which comprises applying an effective amount of a composition as hereinbefore defined to an individual in need thereof. The present invention also provides a method for protecting teeth against dental caries which comprises applying an effective amount of a composition as hereinbefore defined to an individual in need thereof.

The present invention provides a method for the treatment and/or inhibition of dental erosion on a dental surface, comprising contacting the dental surface with a composition as hereinbefore defined.

The present invention provides a method for the treatment and/or inhibition of dental caries on a dental surface, comprising contacting the dental surface with a composition as hereinbefore defined. The invention is further illustrated by the following Examples.

### Test formulations

A dentifrice composition (Formulation 1) as described in Table 1 was prepared as follows:
To a suitable vessel was added purified water, sorbitol and glycerin. Then sodium hydroxide, sodium lactate solution, sodium saccharin, sodium fluoride, potassium nitrate, Gantrez, titanium dioxide, and 20% of the flavour were added and mixed with high shear until solids were dissolved. Whilst mixing under vacuum the dental silica was added, then mixed until wetted out. The cocamidopropyl betaine solution and the remaining 80% of the flavour were added and mixed. Separately in a pre-mix vessel, the xanthan gum was mixed with approximately 95% of the polyethylene glycol to form a slurry. Under vacuum this slurry was added to the main vessel whilst mixing under high shear. The remainder of the polyethylene glycol was added into the pre-mix vessel and the resulting mixture was flushed into the main vessel. The resulting paste was mixed under vacuum until homogenous then transferred to suitable tubes.

**Table 1 - Formulation 1**

| **Ingredient Name** | **%w/w** |
|---|---|
| USP Water | 25.7322 |
| Sorbitol (70% w/w) | 30.0000 |
| Silica (thickening + abrasive) | 17.0000 |
| Glycerin | 8.0000 |
| Potassium Nitrate | 5.0000 |
| Sodium Lactate solution (60%w/w) | 4.1466 |
| Polyethylene Glycol | 3.0000 |
| 47% (aq) Cocamidopropyl Betaine Solution | 2.0940 |
| Gantrez S-97 HSU solution (or ORAREZ W-100 L16) Both (16.5% w/w) | 1.5200 |
| Flavour | 1.2000 |
| Titanium Dioxide | 0.9000 |
| Xanthan Gum | 0.8000 |
| Sodium Saccharin | 0.3000 |
| Sodium Fluoride | 0.2542 |
| Sodium Hydroxide | 0.0530 |
| **Total** | 100.0000 |

| | |
|---|---|
| pH of Formulation 1(1:3 slurry in water) = 6.2 | |

Further formulations (2 to 9) were prepared as follows.

### Formulation 2

Formulation 2 contains no fluoride. The sodium fluoride from Formulation 1 was omitted. Otherwise, the preparation method was identical to that used for Formulation 1.

### Formulations 3 to 9

The preparation method used for Formulations 3 to 9 was identical to that used for Formulation 1. However, as a final step the extra additive(s) (as shown in Table 2 below) were added to the base formulation (Formulation 1). The resulting paste was mixed under vacuum until homogenous then transferred to suitable tubes.

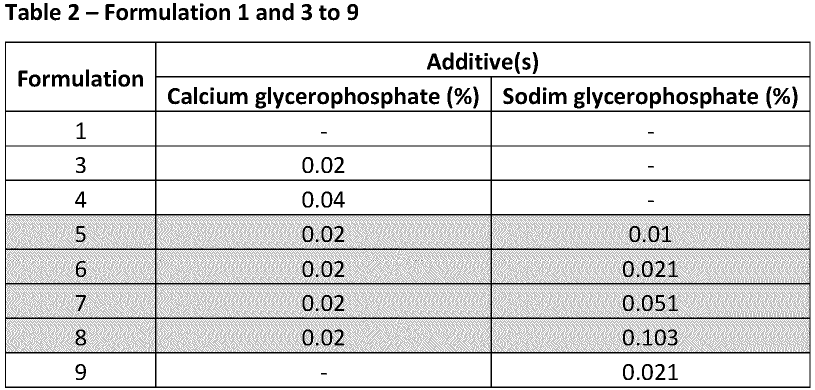

Formulations 5-8 represent formulations of the present invention. Formulas 1-4 and 9 are comparative formulations.

### Test 1 - Study of Lesion Remineralization and Enamel Fluoride Uptake (EFU)

### Introduction

The purpose of this Example was to determine the efficacy of different dentifrices to,
1) promote lesion remineralization;
2) promote enamel fluoride uptake and
3) diminish subsequent demineralization under dynamic conditions simulating *in vivo* caries formation, with dentifrices also tested under different remineralizing conditions.

### Background

pH cycling models were designed to simulate the dynamic variations in mineral saturation and pH associated with the natural caries process (White DJ. The application of in vitro models to research on demineralization and remineralization of the teeth. Adv Dent Res 1995;9(3):175-193).

They reproduce specific events of the caries process under controlled conditions allowing the investigation of individual mechanistic variables that could not be possible to do or would be extremely difficult to do under *in vivo* conditions. At the same time, it is important to recognize that because of not being able to reproduce the whole complexity of caries dynamics, *in vitro* experiments provide only limited information on the effects of different variables on the caries process.

While there are several pH cycling models commonly used, the one developed by White (White DJ. Reactivity of fluoride dentifrices with artificial caries. I. Effects on early carious lesions: Fluoride uptake, surface hardening and remineralization. Caries Res 1987;21:126-140; White DJ. Reactivity of fluoride dentifrices with artificial caries. II. Effects on subsurface lesions: F uptake, F distribution, surface hardening and remineralization. Caries Res 1988;22:27-36) has been shown to be an excellent model to evaluate the remineralization potential of fluoride dentifrices. This model is able to demonstrate a dose-response (0, 250 ppm and 1100 ppm F) in both enamel and dentin (Dunipace AJ, Zhang W, McClure RJ, Stookey GK. An in vitro model for studying human dentin. J Dent Res 1992;71:200; Faller RV. In vitro fluoride dose response below 1100 ppm F (NaF). J Dent Res 1992;71:186; Schemehorn B, Roberts JA, Wood GD. An in vitro remin/demin model showing a fluoride dose response. J Dent Res 1994;73:241) and can be used with either bovine or human enamel (Schemehorn BR, Farnham RL, Wood GD. Fluoride uptake and remineralization in human and bovine enamel. J Dent Res 1992;71:186).*Test Formulations*

The Formulations tested were assigned to groups and were labeled as follows:

**Table 3 - Test Formulations**

| **Group** | **Formulation - Test** |
|---|---|
| A | 1 |
| B | 2 |
| C | 3 |
| D | 4 |
| E | 5 |
| F | 6 |
| G | 7 |
| H | 8 |
| I | 9 |
| J | Control |

### Material and Methods

### Specimen Preparation

Enamel specimens obtained from human permanent teeth were used as the hard tissue test substrate. The teeth were sorted and cleaned. Selection of the tooth for further processing was based on the quality of the enamel and whether the particular tooth surface had sufficient size to obtain a large enough specimen to meet the study requirements. Tooth sections with white spots, cracks and other defects were rejected. The tooth sections were cut into 3 × 3 mm specimens using a Buehler Isomet low-speed saw. The teeth were stored in thymol during the sample preparation process. The 3 × 3 mm specimens were ground and polished to create flat surfaces to facilitate surface microhardness testing using Struers RotoPol 31 / RotoForce 4 polishing unit. The bottom side of the specimens was ground flat to a uniform thickness with 500-grit silicone carbide grinding paper. The topside of the specimen was ground using 1200-grit paper until most of the tooth surface was flattened. The specimens were serially polished using 4000-grit paper followed by 1-mm diamond polishing suspension. Specimens had at least 0.3 mm of enamel thickness. The specimens were sonicated in de-ionized water between each grinding/polishing step. As a final cleaning step, the polished specimens were sonicated in 2 % microliquid. The specimens were assessed with a magnification of 10×. To be acceptable for the study the specimens were required to:
a) not have any obvious cracks or other flaws in the enamel surface;
b) have an evenly polished, high gloss enamel surface;
c) have no contamination on the top surface from sticky wax or any other material.

Each specimen was mounted on the end of an acrylic rod (1/4" diameter x 2" long) using cyanoacrylate (superglue). The sides of each specimen were covered with a varnish so that only the enamel surface was exposed. Eighteen specimens per group were used for this study. Each group will be composed of 3 subgroups of 6 specimens.

### Initial Acid Demineralization

Artificial lesions were formed in the enamel specimens by a 120-hour immersion into a solution of 0.1 M lactic acid and 0.2% Carbopol C907 which was 50% saturated with hydroxyapatite and adjusted to pH 5.0.

### Demineralization (Baseline) Microhardness

Initial hardness of the demineralized specimens was determined using a Vickers microhardness (VMH) indenter at a load of 200 g for 15 seconds. The average specimen microhardness was determined from four indentations on the surface of each specimen. Only specimens with a lesion surface hardness range within 2 standard deviations from the group mean were accepted. Specimens were balanced into groups and subgroups by their post demineralization microhardness values.

### Treatment Phase

### Remineralization Medium

An artificial saliva solution with proteins (2.20 g/L Gastric Mucin, 0.381 g/L NaCl, 0.213 g/L CaCl₂-2H₂O, 0.738 g/L KH₂PO₄, 1.114 g/L KCl, pH 7.0) was used as the remineralization medium in all treatment regimens. Fresh artificial saliva was used each day (changed during the acid challenge period).

### Treatment Regimen

The cyclic treatment regimen consisted of a 4 h/day acid challenge in the lesion forming solution and four, one-minute treatment periods.

Treatments were prepared by adding 5.0 g of toothpaste to 10 mL of fresh artificial saliva in a beaker with a magnetic stirrer. A fresh treatment for each subgroup was prepared prior to each treatment. All treatments were stirred at 350 rpm.

After the treatments, the specimens were rinsed with running deionized water. All specimens were then placed back into artificial saliva. The remaining time (~20 hours) the specimens were in the artificial saliva, remineralization system. The regimen was repeated for 20 days for the groups tested with artificial saliva with proteins. The treatment schedule for this experiment is given in the table below:

**Table 4 - Treatment Schedule**

| | |
|---|---|
| 8:00-8:01 a.m. | Dentifrice Treatment^{a} |
| 8:01-9:00 a.m. | Artificial Saliva |
| 9:00-9:01 a.m. | Dentifrice Treatment |
| 9:01-10:00 a.m. | Artificial Saliva |
| 10:00 a.m.-2:00 p.m. | Acid challenge |
| 2:00-3:00 p.m. | Artificial Saliva |
| 3:00-3:01 p.m. | Dentifrice Treatment |
| 3:01-4:00 p.m. | Artificial Saliva |
| 4:00-4:01 p.m. | Dentifrice Treatment |
| 4:01 p.m.-8:00 a.m. | Artificial Saliva |

| | |
|---|---|
| ^{a} *On the first day, this treatment was not given; the test began with one hour in artificial saliva to permit pellicle development prior to any treatments.* | |

### Post Treatment Microhardness

The average specimen microhardness was determined, as described above, from four indentations on the surface of each specimen, next to the baseline indentations. The difference between the hardness following treatment and initial lesion hardness indicated the ability of that treatment to enhance remineralization after 20 days of treatments.

### Fluoride Analysis

At the end of the 20-day treatment regimen, the fluoride content of each enamel specimen was determined using the microdrill technique to a depth of 100µm. The diameter of the drill hole was determined. The enamel powder from the drill hole was collected, dissolved (20µl of HClO₄, 40µl Citrate/EDTA Buffer and 40µl DI water) and analyzed for fluoride by comparison to a similarly prepared standard curve. Fluoride data was calculated as µg F/cm³: (µg F X dilution factor - volume of drilling).

### Determination of Enamel Resistance to Demineralization

The resistance of the treated enamel to a subsequent acid challenge was determined by placing the treated specimens into the lesion formation solution (with no remineralization phase) for one 2-hour period of simulated plaque acid challenge (SPAC).

### Post SPAC Microhardness

Following the acid challenge, the surface hardness of the specimens was measured again. The average specimen microhardness was determined from four indentations on the surface of each specimen, next to the baseline indentations. The change in hardness between the subsequent demineralization and the baseline measurements was calculated. The change reflected the degree of resistance to demineralization provided by each treatment.

### Data Management and Analysis

The mean, SD (standard deviation), and SEM (standard error of the mean; the SD divided by the square root of the sample size) of each parameter for each group was calculated. Statistical analysis of the data was performed with a one-way analysis of variance models using SPSS Software. Additionally, all pair wise comparisons between the groups were conducted (Student Newman Keuls test).

### Results and Conclusions

The results are shown in the tables below.

### Test 1 Microhardness (VHN) (Table 5)

There was no significant difference between Formulations 8, 6, and 9; these Formulations were the most effective in promoting remineralization after 20 days of pH cycling. Formulation 7 was less effective than the above Formulations but more effective than the remaining Formulations. There was no significant difference between Formulations 3, 5, 1 and 4. Formulations 3 and 5 were more effective than Formulation 2. There was no significant difference between Formulations 1, 4, and 2.

**Table 5: Test 1 Microhardness (VHN) Data Summary**

| **Group** | **Formulation** | | **Baseline VHN** | **Post 20-day VHN** | **Δ 20-day** | **VHN ₙ** |
|---|---|---|---|---|---|---|
| H | 8 | Mean | 41.1 | 98.0 | 56.9 | |
| | | SD | 6.4 | 20.7 | 20.1 | 18 |
| | | SEM | 1.5 | 4.9 | 4.7 | |
| F | 6 | Mean | 41.0 | 96.7 | 53.8 | |
| | | SD | 6.5 | 18.5 | 13.6 | 17* |
| | | SEM | 1.5 | 4.4 | 3.3 | |
| I | 9 | Mean | 41.1 | 92.2 | 51.0 | |
| | | SD | 6.3 | 22.5 | 18.8 | 18 |
| | | SEM | 1.5 | 5.3 | 4.4 | |
| G | 7 | Mean | 41.1 | 85.6 | 41.9 | |
| | | SD | 6.3 | 16.2 | 10.6 | 17* |
| | | SEM | 1.5 | 3.8 | 2.6 | |
| C | 3 | Mean | 41.2 | 69.1 | 25.8 | |
| | | SD | 6.9 | 15.7 | 12.9 | 17* |
| | | SEM | 1.6 | 3.7 | 3.1 | |
| E | 5 | Mean | 41.1 | 67.9 | 23.7 | |
| | | SD | 6.8 | 18.3 | 10.2 | 17* |
| | | SEM | 1.6 | 4.3 | 2.5 | |
| A | 1 | Mean | 41.1 | 62.0 | 20.8 | |
| | | SD | 7.1 | 12.4 | 9.3 | 18 |
| | | SEM | 1.7 | 2.9 | 2.2 | |
| D | 4 | Mean | 41.1 | 63.3 | 20.5 | |
| | | SD | 6.8 | 13.5 | 8.6 | 17* |
| | | SEM | 1.6 | 3.2 | 2.1 | |
| B | 2 | Mean | 41.1 | 52.7 | 11.6 | |
| | | SD | 7.1 | 9.2 | 6.6 | 18 |
| | | SEM | 1.7 | 2.2 | 1.6 | |

| | | | | | | |
|---|---|---|---|---|---|---|
| Groups within lines were not significantly different (p>0.05) *Data were rejected as outlier data | | | | | | |

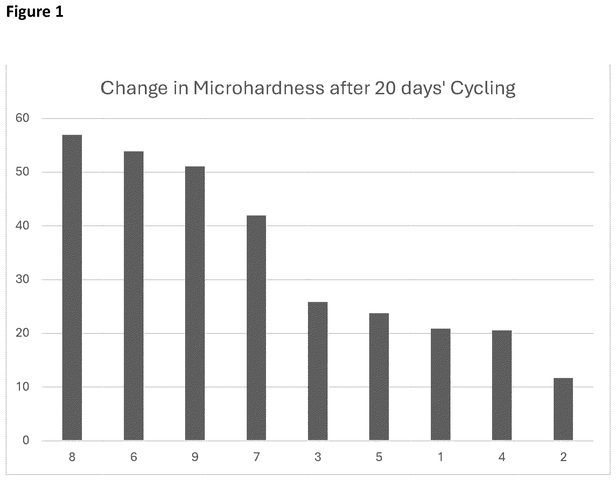

(These results are also shown pictorially in Figure 1)

### Test 1 Enamel Fluoride Uptake (Table 6)

There was no significant difference between Formulations 8 and 7; these Formulations were the most effective in promoting enamel fluoride uptake. There was no significant difference between Formulations 9, 6, 1, 5, 3, and 4. Formulation 2 was the least effective in promoting enamel fluoride uptake.

**Table 6: Test 1 Fluoride Data Summary**

| **Group** | **Formulation** | | **EFU (ppm)** | |
|---|---|---|---|---|
| | | | | n |
| H | 8 | Mean | 828 | |
| | | SD | 105 | 17* |
| | | SEM | 25 | |
| G | 7 | Mean | 747 | |
| | | SD | 181 | 18 |
| | | SEM | 43 | |
| I | 9 | Mean | 630 | |
| | | SD | 154 | 17* |
| | | SEM | 37 | |
| F | 6 | Mean | 629 | |
| | | SD | 132 | 17* |
| | | SEM | 32 | |
| A | 1 | Mean | 589 | |
| | | SD | 163 | 18 |
| | | SEM | 38 | |
| E | 5 | Mean | 545 | |
| | | SD | 108 | 17* |
| | | SEM | 26 | |
| C | 3 | Mean | 506 | |
| | | SD | 189 | 17** |
| | | SEM | 46 | |
| D | 4 | Mean | 503 | |
| | | SD | 104 | 18 |
| | | SEM | 25 | |
| B | 2 | Mean | 33 | |
| | | SD | 9 | 18 |
| | | SEM | 2 | |

| | | | | |
|---|---|---|---|---|
| Groups within lines were not significantly different (p>0.05) *Data were rejected as outlier data **Data not obtained from one specimen due to lost powder | | | | |

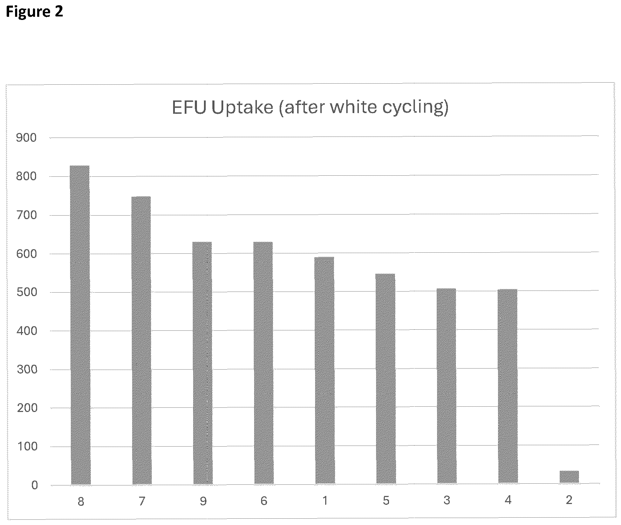

(These results are also shown pictorially in Figure 2)

There was no significant difference between Formulations 3, 1, and 7 in promoting enamel fluoride uptake. Formulation 2 was the least effective.

In summary the results indicate that a combination of water, lactic acid or salt thereof, a copolymer, NaF, and both Calcium glycerophosphate and Sodium glycerophosphate in the Formulations tested more effectively promotes enamel fluoride uptake with the consequential improvement in enamel microhardness.

Particularly effective formulas are 7 and 8, wherein the ratio of calcium glycerophosphate to sodium glycerophosphate is between about 1:2.5 and 1:5 by weight.

### Test 2 - Enamel Erosion (Featherstone Model pH Cycling Testing Study)

### Introduction

In order to evaluate the effectiveness of the test formulation a clinical study was performed to compare the effectiveness of the test formulations against a fluoride-free placebo control for enamel erosion.

### Procedure

Prepared caries-free human enamel specimens were subjected to a pH cycling regimen for 14 days, each day consisting of two dentifrice treatments, a period of demineralization and a period of remineralization. There were two weekend periods when samples were placed in remineralization solution at 37°C At the end of the 14-day pH cycling treatment regimen, the specimens were evaluated via published cross-sectional microhardness techniques for determination of subsurface % mineral loss/gain.

### Specimen Preparation

Caries free human teeth (erupted third molars, molars and pre-molars) were collected and carefully inspected for cracks and surface imperfections. An area approximately 4x5mm was marked in pencil on the surface of the tooth where there were no discernible cracks or surface imperfections. The roots were cut off from the tooth and the crown was cut in half to isolate the selected surface that was void of cracks and surface imperfections. The half that was not marked was discarded. The enamel surface was then lightly abraded with 600 grit, flat silicon carbide wet/dry grinding paper (Buehler), following the shape of the tooth for 30 seconds to remove any surface debris or stain. The teeth were then inspected again for surface imperfections and cracks. An area approximately 4x5mm was marked again (note: if there was not a 4 × 5 mm area on the tooth that was free of cracks and imperfections after sanding, the tooth was discarded). The specimens were randomly placed into treatment groups (N=10). The entire enamel surface except for the one area measuring approximately 4x5mm on a flat, clean surface of the enamel was covered with an acid resistant nail polish (Cover Girl Red Revolution). This created an exposed area for testing and the remaining enamel was controlled and not subjected to the cycling process. Once the specimen window had been created, it did not come in contact with any surfaces throughout the entire process.

Each group of N=10 specimens was attached to plastic rods cut to a length of approximately five and a half inches. The cut surface of the tooth was attached to one side of the thin rod with the open window facing away from the rod. Five specimens were attached to each side stacked one after the other from the bottom. Hot glue was used to fill in any spaces that occurred between the 10 teeth per rod. During the cycling process, specimens were treated collectively by treatment group and were suspended so that the enamel was exposed to the designated solution continuously. Specimen rods were stored suspended within a sealed jar under damp conditions until testing began.

### Solution Preparation

Remineralization and demineralization solutions were prepared twice a week as stock, according to the following procedures. Solutions were dispensed into individual containers at the beginning of the week and used for 2-3 days. Unused stock solutions of the de-/remineralization solutions were stored in sealed containers at room temperature. Midweek, used solutions were discarded and fresh stock solution was dispensed into the individual containers for the remainder of the week. At the end of the week, fresh remineralization solution was prepared and dispensed for the weekend (2 day) remineralization period. The following week, stock solutions were again prepared and dispensed as described. Special attention was given to the preparation of the re-/demineralization solutions (pH, ingredient source, volumes, glass/plastic ware etc).

### Demineralization Solution - 4 L

17.24 mL (18.2g) of Glacial Acetic Acid
1.088 grams of dibasic, anhydrous, Calcium Phosphate powder

Approximately 3800mL of DI water was added to a 4L beaker. The Glacial Acetic Acid was added and allowed to stir for 2 minutes. The Calcium Phosphate was then added and allowed to completely dissolve while stirring. The pH was then adjusted to 4.5 with 50% NaOH. The solution was then transferred to a 4L Volumetric flask and the volume was increased to 4L. (This solution had a shelf life of 7 days)

### Remineralization Solution - 4 L

1 0.6250g of Dibasic Potassium Phosphate
2 1.4200g of Calcium Nitrate 4-hydrate
3 44.8000g of Potassium Chloride
4 16.74g of BisTris (Bis-tris methane)

All of the components were dissolved separately in 100mL of DI water. The dissolved solutions were then added one at a time in the order given above to approximately 4L of DI water with a 10-minute wait period between additions. The solution was then adjusted to a pH of 7.0 with concentrated HCl and brought to 4L. Care was taken not to overshoot the pH. If any precipitates form or if the pH was overshot during the making of this solution, it was discarded and prepared again. (This solution had a shelf life of 7 days)

### Treatment Groups and Test Sample ID's

The test samples were coded. Formulations 1 to 9 were used.

### Treatment Regimen

The treatment regimen was a 24-hr period that was repeated for a total of 14 treatment days, interrupted by two weekends when specimens were stored at 37 OC in remineralizing solution (total nearly 3-week period). On the first day of the study (a Monday) the following procedure was used:
1) One dentifrice slurry (1:3 w/w) was prepared for each dentifrice sample mixing 1-part by weight dentifrice with 3-parts parts by weight DI H2O into a 50 ml beaker with a Teflon coated stir bar. The slurry was mixed on a nonaerating mixer for 4-5 minutes at a speed fast enough to completely disperse the paste without causing aeration. The total volume of the slurry equalled approximately 4ml/tooth specimen. The slurry was poured into a designated FALCON 50 mL polypropylene conical tube (352070). The specimens on the rods were immersed in the slurry for a 1-minute period with occasional hand agitation. The slurries were made fresh just prior to each treatment throughout the cycling process.
2) After the 1-minute treatment, the specimens were removed from the slurry and rinsed with DI H2O. Treatment slurries were discarded. Specimens were then placed in demineralizing solution described previously (pH 4.5). Each treatment rod of N=10 specimens was immersed in 400 ml of demineralization solution (40 mL/tooth) in an individual 16 oz high density polyethylene sample jar (Qorpak 7356G). A designated jar was used for each treatment group to insure no cross contamination occurred between treatments. All specimens were completely submerged in the solution and placed in an incubator at 37° C without stirring for a period of 6-hours. A small hole was drilled into the lid of the sample jar and the sample rod was inserted into the hole to facilitate the suspension of the samples in the demineralizing solution during the incubation period. The lids of the jars were secured to prevent evaporation. The demineralizing solution was obtained fresh from stock daily. A new batch of demineralizing solution was prepared at the beginning of each 5-day treatment period, as described above.
3) After the 6-hour demineralizing period, the specimen jars were removed from the incubator and placed at room temp. Preparations are made to initiate a second treatment for the day. After fresh slurries were prepared (as outlined above), the specimen rods were removed from demineralizing solution, rinsed in DI H2O, and immersed for 1-minute in the slurry of the assigned test product in a disposable 50 mL conical centrifuge tube (as described in step 1).
4) After the 1-minute treatment period, the specimens were rinsed with DI H2O. Each treatment group of N=10 specimens was immersed in 200 ml of remineralization solution (20 ml/tooth). An individual 8 oz high density polyethylene sample jar (Qorpak 7355G) was used for each treatment group to ensure no cross contamination occurred between treatments. All specimens were completely submerged in the solution and placed in an incubator at 37° C without stirring overnight (16-18 hrs). Sample rods were incorporated into the lids of the jars and capped to prevent evaporation as described in step 2. This solution approximated the degree of saturation with respect to hydroxyapatite in saliva and is similar to that described by ten Cate and Duijsters (ten Cate JM, Duijsters PPE. Demineralization and remineralization of artificial enamel lesions. Caries Res 1982;16:201-210.). The remineralizing solution was obtained fresh from stock daily except weekends. A fresh stock batch of solution was prepared again on the beginning of the next treatment leg.

On the morning of day 2 through day 5, steps 1-4 are repeated.

A tabular Representation of Conventional Treatment Regimen follows:-

**Table 7 - Treatment Regimen**

| **Treatment Day** | **Time** |
|---|---|
| Rinse | |
| Treatment 1 | 1 minute |
| Rinse | |
| Demineralization | 6 hours |
| Rinse | |
| Treatment 2 | 1 minute |
| Rinse | |
| Remineralizing solution | 16-18 hours |

### Weekend Remineralizing Period

On the afternoon of day 5, following the second treatment period and deionized water rinse, specimens were placed in a freshly prepared batch of remineralizing solution. Each treatment rod of N=10 specimens was immersed in 200 mL of remineralization solution (20 mL/tooth). A separate individual 8 oz high density polyethylene sample jar was used for each treatment group to ensure no cross contamination occurred among treatments. All specimens were completely submerged in the solution, the jars capped to prevent evaporation and placed in an incubator at 37 °C without stirring until the first treatment on Monday morning.

The second week of the study began with removing the specimen jars from the 37 °C incubator, rinsing with deionized water and beginning the treatment regimen as described on Day 1. The same schedule continued throughout the week concluding with the remineralizing period described previously for the weekend. By the end of the second week, the enamel specimens had been treated for 10 of the total 14-days.

The third week of the study began with removing the specimen jars from the 37 °C oven, rinsing with deionized water and beginning the treatment regimen as described on Day 1. The schedule continued for four complete days of cycling/treatments. On the morning of the fifth day, the specimens were removed from the remineralization solution. By this time, the enamel specimens had been treated for 14 of the required 14 days of cycling. Specimens were rinsed thoroughly with deionized water and the specimens on the rods were stored suspended within a sealed jar under damp conditions until evaluation of the teeth began.

### Specimen Mounting and Lesion Analysis

After 14-days of pH cycling, specimens were sectioned longitudinally, through the lesion and mounted. The specimens of each treatment rod (two per group) were mounted five per block with cold set acrylic resin covering all surfaces except the cut face. The cut faces were sanded with 600 grit sandpaper to remove any acrylic that may have gotten on the tooth surface during the mounting process. This also flattened the block. The blocks were then polished serially with 9, 3, and 1 µm lapping film. A high luster was needed to permit visualization of microhardness indents. Cross-sectional microhardness assessments were then conducted by first identifying the enamel surface of the specimen under microscopy, moving the stage 12.5 µm deep into the enamel lesion and making a hardness indentation using a Knoop diamond indenter with a load of 10 gF and a dwell time of 15-seconds. This same procedure was repeated at 25, 37.5, 50, 62.5 and 75 µm deep into the enamel. Additional indentations were made at 100, 125, 150, 175, 200, 225, 250, 275 and 300 µm deep into the enamel with a load of 50 gF weight and a dwell time of 15-seconds. These procedures were a slight modification of those described in Featherstone et al., 1983 (Featherstone JDB, O'Reilly MM, Shariati M, Brugler S. Enhancement of remineralization in vitro and in vivo. In: Leach SA. Factors relating to demineralisation and remineralisation of the teeth. Oxford: IRL Press Ltd, 1986;23-34) and ten Cate et al (as above).

### Calculations

Microhardness: The length (diagonal) of the Knoop indentations used to calculate surface microhardness using the equations per the instrument/software based on the specified conditions above.

Volume % Mineral: Featherstone, et al (1983), studied the relationship between Knoop hardness number (KHN) and volume percent mineral and provided the following equation:$√ \left(KHN\right) = 0.197 \left[volume percent mineral\right] - 0.24$

Calibration of microhardness testers: Sound enamel is generally assigned a value of 85 volume % mineral. That is, by volume, enamel consists of about 85% mineral and 15% organic or other materials. Knowing this value, one can take several indents in sound enamel and insert the observed indent length to create a calibrated equation for their own hardness tester, rather than simply accepting the equation and constants given in the 1983 Featherstone publication. Volume percent mineral is directly proportional to 1/L, where L is the indent length. The microhardness tester was calibrated by conducting several hundred sound enamel hardness intent and measuring the resulting lengths/diagonals. The overall mean indent length was substituted into the equation, using the accepted value of 85 volume % mineral for sound enamel. Because the indents made at 12.5, 25, 37.5, 50, 62.5, and 75 µm were made using a 10g load, a constant was also calculated for the 10g load in the same manner. $C = L * 85 % / wt \frac{1}{2}$ Where:
85% is the accepted value for sound enamel
C = constant
Wt = force used to create the indents in grams (50g or 10g)
L = the length of the indent in sound enamel

Calculation of Volume % Mineral from an indent length: In order to convert an indent length into volume percent mineral, Equation 1 was utilized. All indent lengths made in specimen samples were converted to volume % mineral.

### Area Under the Curve (AUC) and Delta Z

All volume % mineral values collected were normalized using the underlying sound enamel. That is, volume % mineral values at 150, 175, 200, 225, 250, 275 and 300 µm will be used to calculate an average volume % mineral for sound enamel. Each volume % mineral value in a series was normalized so that the average of 150 through 300 µm was 85%. After normalization, the area under the curve (AUC) was calculated using the trapezoid method. The trapezoid method calculates the AUC using the average volume % mineral value of two adjacent indents and the distance between the indents. The AUC was then subtracted from that of sound enamel (85 volume percent mineral x [300 - 12.5 µm] = 24,437.5) to achieve a mineral loss compared to sound enamel or Delta Z Value for each series of indents. Two (2) series of indents were collected for each specimen, averaged and resultant Delta Z Value calculated for each specimen.

### Data Reporting and Analyses

The data is summarized in Table I below. All individual specimen Delta Z values are included and the Mean (N=10) standard deviation (SD) and standard error (SEM) were calculated for each treatment group. Statistical analyses were performed by a One-Way Analysis of Variance (ANOVA) and Student-Newman-Keuls (SNK) multiple comparison model using Sigma Plot (14.5) Software. Statistical significance was determined at the 95% Cl (p < 0.050).

### Results and Conclusions

Treatment group 2 (Delta Z = 5492.39 ± 366.93 | Mean ± SEM) exhibited a significantly greater (p ≤ 0.001) volume % mineral loss (enamel demineralization) following the 14-day in vitro pH cycling treatment regimen compared to all other test samples in this assessment. Treatment groups 6 (Delta Z = 39.17 ± 100.96) and 7 (Delta Z = 44.11 ± 85.60) both exhibited significantly a lesser (p < 0.050) volume % mineral loss, indicative of more effective prevention of enamel demineralization, compared to treatment groups 4 (Delta Z = 469.71 ± 83.78) and 3 (Delta Z = 381.25 ± 73.08) following the 14-day in vitro treatment regimen. The reaming treatment groups exhibited Mean (N=10) Delta Z values (volume % mineral loss compared to sound enamel) ranging from Delta Z = 356.27 (5) to Delta Z = 97.13 (8) and no additional statistically significant differences (p > 0.050) in efficacy were observed between test samples in this in vitro assessment.

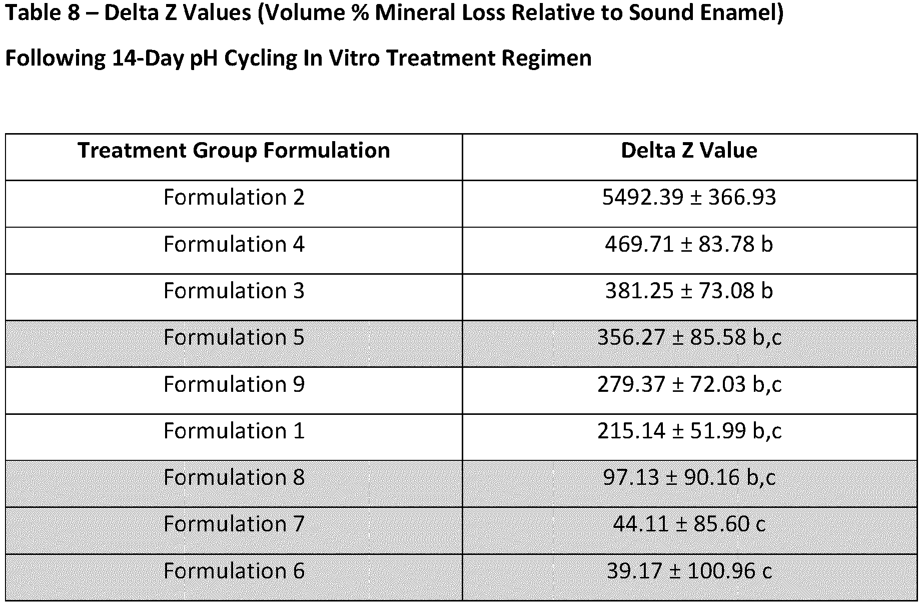

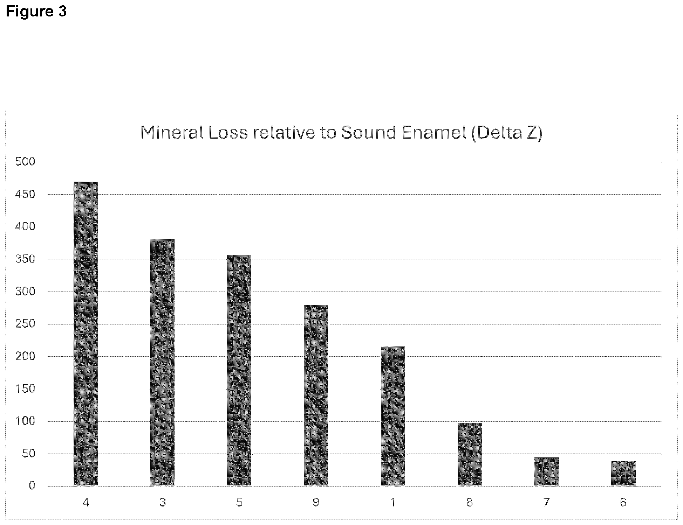

(These results are also shown pictorially in Figure 3)

Mean (N=10) ± SEM
Greater Delta Z values indicate increased enamel volume % mineral loss (demineralization).

The results show that formulation 6 to 8 exhibited significantly less volume % mineral loss, indicative of more effective prevention of enamel demineralization.

In summary the results indicate that a combination of Calcium glycerophosphate and Sodium glycerophosphate in Formulations more effectively promotes remineralisation and enamel fluoride uptake in lesioned enamel and prevents better demineralisation of sound enamel compared formulations which do not include Calcium glycerophosphate and Sodium glycerophosphate.

## Claims

1. A dentifrice composition comprising:
a) water, between 20 % and 40 % by weight,
b) lactic acid or a salt thereof, between about 0.1 % and about 5 % by weight,
c) a copolymer of methyl vinyl ether (MVE) with maleic anhydride or acid, between about 0.1 % and about 0.5 % by weight,
d) sodium fluoride
e) calcium glycerophosphate and sodium glycerophosphate between about 0.01 % and about 0.50 % by weight;
and wherein the dentifrice composition has a slurry pH in the range from greater than about 5.5 to less than about 6.5.

2. The dentifrice composition of claim 1 wherein the calcium glycerophosphate comprises about 0.01 % to about 0.1 % by weight and the sodium glycerophosphate comprises about 0.01 % to about 0.25 % by weight.

3. The dentifrice composition any of the previous claims wherein the calcium glycerophosphate comprises about 0.015 % to about 0.025 % by weight and the sodium glycerophosphate comprises 0.02 % to 0.20 % by weight;

4. The dentifrice composition of any of the previous claims wherein the calcium glycerophosphate comprises about 0.015 % to about 0.025 % and the sodium glycerophosphate comprises about 0.04 % to about 0.12 % by weight.

5. The composition of any of the previous claims wherein the calcium glycerophosphate comprises about 0.02 % by weight and the sodium glycerophosphate comprises about 0.05 % by weight; or wherein the calcium glycerophosphate comprises about 0.02 % by weight and the sodium glycerophosphate comprises about 0.1 % by weight.

6. The dentifrice composition according to any of the previous claims wherein the lactic acid is provided as sodium lactate.

7. The dentifrice composition according to any of the previous claims wherein the composition has a slurry pH in the range from 6.0 to 6.5.

8. The dentifrice composition according to any of the previous claims wherein the copolymer is a copolymer of MVE with maleic acid.

9. The dentifrice composition according to claim 8 wherein the copolymer is a 1:1 copolymer of MVE with maleic acid.

10. The dentifrice composition according to any of the previous claims wherein the copolymer has a molecular weight in the range 100,000 to 2,000,000.

11. The dentifrice composition according to any one of claims 1 to 13 further comprising a desensitising agent.

12. The dentifrice composition according claim 11 wherein the desensitising agent is potassium nitrate.

13. The dentifrice composition as claimed in any one of claims 1 to 12 for use in protecting teeth against dental erosion.

14. A composition as claimed in any one of claims 1 to 12 for use in protecting teeth against dental caries.
